# FASCICULE DE BREVET EUROPEEN

(11) **EP 0 349 435 B1**
(45) Date de publication et mention de la délivrance du brevet: **24.05.1995**
(21) Numéro de dépôt: 89401871.2
(22) Date de dépôt: 29.06.1989
(51) Int. Cl.: C12P 21/02, C12N 15/81, C12N 1/18

(54) **Procédé de préparation d'une protéine par des levures utilisant un système inductible, vecteurs et souches transformées correspondantes**
Verfahren zur Herstellung eines Proteins durch Hefe mit Hilfe eines induzierbaren Systems, Vektoren und entsprechende transformierte Stämme
Method for the preparation of a protein by yeast using an inductible system, vectors and corresponding transformed strains

(30) Priorité: 01.07.1988 FR 8808978
(43) Date de publication de la demande: 03.01.1990
(73) Titulaire: CENTRE NATIONAL DE LA RECHERCHE SCIENTIFIQUE (CNRS), 75794 Paris Cédex 16 (FR)
(72) Inventeur: Chambon, Pierre Henry, F-67113 Blaesheim (FR); Metzger, Daniel, F-67400 Illkirch-Graffenstaden (FR); White, John, F-67000 Strasbourg (FR)
(74) Mandataire: Warcoin, Jacques

(56) Documents cités:
- NATURE, vol. 334, 7 juillet 1988, pages 31-36, Londres, GB; D. METZGER et al.:"The human oestrogen receptor functions in yeast"
- CELL, vol. 52, no. 2, 29 janvier 1988, pages 169-178, Cell Press, Cambridge, Massachusetts, US; N. WEBSTER et al.: "The yeast UASg is a transcriptional enhancer in human HeLa cells in the presence of the GAL4 trans-activator"
- CELL, vol. 52, 29 janvier 1988, pages 161-167, Cell Press, Cambridge, Massachusetts, US; H. KAKIDANI et al.: "GAL4 activates gene expression in mammalian cells"
- BIOTECHNOLOGY, vol. 4, no. 8, août 1986, pages 726-730, New York, US; T.ETCHEVERRY et al.: "Regulation of the chelatin promoter during the expression of human serum albumin or yeast phosphoglycerate kinase in yeast"
- CELL, vol. 51, no. 1, octobre 1987, pages 113-119, Cell Press, Cambridge, Massachusetts, US; J. MA et al.: "A new class of yeast transcriptional activators"
- CELL, vol. 52, no. 2, 29 janvier 1988, pages 179-184, Cell Press, Cambridge, Massachusetts, US; K. LECH et al.: "DNA-bound fos proteins activate transcription in yeast"
- CELL, vol. 56, 10 février 1989, pages 335-344, Cell Press, Cambridge,Masachussetts, US; M. BEATO: "Gene regulation by steroid hormones"
- Biochemical and biophysical research communications, vol.178, no.3 (1991) pp.1167-1175.

## Description

L'invention concerne des systèmes d'expression inductibles, les souches transformées correspondantes et leurs méthodes d'obtention pour produire des protéines, notamment des protéines hétérologues, dans les levures.

La technique de l'ADN recombinant permet d'exprimer des gènes de protéines hétérologues dans les levures. Ainsi, la construction de vecteurs renfermant des séquences promoteurs de levure correspondant aux gènes d'enzymes glycolytiques de Saccharomyces cerevisiae tels que la 3-phosphoglycérate kinase (PGK), une alcool déshydrogénase (ADH1) ou la glycéraldéhyde 3 phosphate déshydrogénase (GPD), a permis de produire des protéines intéressantes par fusion de leur séquence codante au promoteur de levure, par exemple l'interféron leucocytaire selon Hitzeman R.A. et al. Nature 293, 717-722 (1981) ou l'antigène de surface de l'hépatite B selon Bitter G.A. et al. Gene 263-274 (1984).

Dans de nombreux cas, il a été observé que la protéine hétérologue produite est toxique pour la cellule hôte et provoque l'instabilité des plasmides et la sélection de cellules qui n'expriment plus la protéine concernée. Des méthodes permettant de réprimer l'expression du gène de la protéine pendant la phase de croissance cellulaire, puis d'induire une production élevée de la protéine pendant le stade final de culture, ont été trouvées en utilisant un promoteur régulé par un changement en sources carbonées, par exemple le promoteur du gène de la galactokinase (GAL1) ou le promoteur du gène de l'uridine diphosphoglucose 4-épimérase (GAL10) qui correspondent à deux des quatre gènes responsables de l'utilisation du galactose chez la levure et dont l'expression est réprimée par le glucose et induite par le galactose. Des vecteurs comprenant le promoteur GAL1 lié à un gène hétérologue ont été décrits, permettant de faire croître la levure dans un milieu contenant du glucose, puis de faire exprimer la protéine quand le galactose est présent dans le milieu. Des souches de levure transformées avec de tels vecteurs sont décrites, par exemple dans la demande de brevet GB 2 137 208 qui, sous le contrôle du promoteur GAL1, expriment en présence de galactose des protéines telles que l'hormone de croissance bovine ou la prorénine. La plupart des promoteurs de levure renferment en amont d'éléments TATA qui médient l'initiation de la transcription, des éléments régulateurs de la transcription "upstream activating sequence" (noté UAS) qui représentent le site de fixation de protéines régulatrices de la transcription, tels que GAL4 pour l'activation des gènes du métabolisme du galactose, elles-mêmes sous le contrôle de leurs propres promoteurs.

Webster et al. (Cell, Vol. 52, No. 2, pages 169-178, 1988) décrit la stimulation de la transcription de gènes dans des cellules humaines HeLa par la protéine transactivateur de levure GAL4, sous le contrôle d'un promoteur d'eucaryotes supérieurs comprenant une séquence régulatrice UAS_{G} de levure ainsi que par un transactivateur chimère GAL4-domaine de fixation de l'ADN du récepteur oestrogène humain hER, sous le contrôle d'un promoteur d'eucaryotes supérieurs comprenant une séquence humaine "estrogène-responsive enhancer element" ERE.

Différents moyens ont déjà été proposés pour améliorer l'efficacité de transcription de gènes hétérologues dans les levures. En particulier, il a été proposé d'utiliser des promoteurs hybrides qui comportent, en amont de l'élément TATA d'un promoteur constitutif de levure, une séquence régulatrice UAS extrinsèque, telle que UASg qui correspond à la séquence intergénique GAL1-GAL10 et que l'on insère par exemple en amont de l'élément TATA d'un promoteur constitutif de levure tel que GPD, comme cela est décrit dans la demande de brevet WO 86/00680, ou tel que PGK, comme cela est décrit dans la demande de brevet EP 258 067. Les séquences UASg insérées permettent successivement de réprimer puis d'exprimer le promoteur constitutif par la source de carbone utilisée telle que le glucose comme répresseur et le galactose comme inducteur.

Cependant, la mise en oeuvre des promoteurs en général ou de tout promoteur hybride implique une maîtrise difficile à réaliser des niveaux totalement réprimés ou induits parce qu'ils font intervenir un ensemble de protéines régulatrices de la transcription. Ainsi, le gène GAL4 active la transcription par la présence de galactose qui est directement ou indirectement responsable de la dissociation d'un complexe entre GAL4 et une protéine antagoniste GAL80, tandis que d'autres protéines interviendraient dans la répression par le glucose, telles que GAL82 et GAL83 selon Yocum R.R. et al., MBC 4 1985-1998 (1984).

En plus des inconvénients évoqués précédemment, ce type de systèmes inducteurs au galactose et/ou répresseurs au glucose ne sont pas très polyvalents, en effet, ils ne peuvent pas être utilisés pour des levures qui n'assimilent pas le glucose et/ou le galactose et ne peuvent pas être utilisés dans le cas où l'un de ces éléments constituent tout ou partie du substrat carboné du milieu de culture.

Il est donc intéressant de disposer chez les levures de systèmes inducteurs pouvant être induits par des produits qui ne sont pas, en eux-mêmes, nécessaires à la culture de ces cellules, c'est le cas par exemple des hormones, notamment des hormones stéroïdiennes.

C'est pourquoi, la présente invention concerne un procédé de préparation d'une protéine par des levures, selon lequel :
- on cultive des cellules de levure qui contiennent :
   . une séquence d'ADN codant pour ladite protéine sous le contrôle d'éléments assurant son expression dans les levures, lesdits éléments comprenant une séquence de contrôle de transcription inductible par un complexe formé par un récepteur et un ligand,
   . une séquence d'ADN fonctionnelle dans la levure codant pour ledit récepteur, le récepteur comprenant deux parties essentielles dont l'une reconnaît le ligand de façon à former un complexe avec ledit ligand et l'autre se fixe sur ladite séquence contrôle de transcription, la partie du recepteur qui reconnaît le ligand est de préférence d'origine eucaryote supérieure et le ligand n'est pas nécessaire à la culture des cellules mais est susceptible de pénétrer dans lesdites cellules lorsqu'il est ajouté au milieu de culture,
- on ajoute ledit ligand dans le milieu de culture à un moment convenant pour l'induction,
- on récupère la protéine synthétisée.

L'expression des protéines par des levures utilisant les techniques des ADN recombinants est considérée comme étant bien connue de l'homme de métier. Un nombre considérable de publications ont déjà décrit la préparation de protéines, notamment de protéines hétérologues, grâce à des levures en utilisant des vecteurs d'expression. Ces vecteurs d'expression comportent la plupart du temps, outre la séquence codant pour ladite protéine, les éléments contrôlant son expression dans les levures, c'est-à-dire en général un promoteur et un terminateur.

Dans la plupart des cas, ces vecteurs sont des vecteurs non intégratifs, c'est-à-dire des plasmides, ils comportent alors une origine de réplication efficace dans les levures, notamment l'origine du plasmide 2» ou une séquence ars propre à ladite levure.

Ces vecteurs d'expression non intégratifs comportent, en outre, des éléments permettant d'assurer leur maintien dans les cellules, soit en utilisant comme marqueur de sélection un gène de résistance ou bien un élément complémentant une auxotrophie de la souche hôte URA3 ou LEU2 par exemple.

Dans certains cas, et afin de pallier les inconvénients liés à l'utilisation de vecteurs à autoréplication, on a pu développer des vecteurs assurant l'intégration des séquences en cause au niveau chromosomique. Ce type de vecteur permet d'obtenir une souche plus stable mais l'amplification au niveau de l'expression est parfois plus faible qu'avec un plasmide non intégratif.

Les vecteurs d'intégration comportent la plupart du temps au moins une séquence homologue d'une séquence chromosomique qui assurera l'échange et l'intégration.

Il doit être compris que la présente invention portant essentiellement sur l'induction de la transcription, les vecteurs d'expression qui seront mis en oeuvre peuvent être aussi bien de type intégratif que non intégratif.

Bien que le procédé en cause soit plus particulièrement destiné à la préparation de protéine hétérologue, il est possible de l'utiliser pour exprimer des protéines de levures, notamment dans le cas de systèmes assurant l'hyperexpression d'un gène qui peut conduire dans certains cas à la mort des cellules avant que l'optimum de biomasse soit obtenu.

Par "protéine hétérologue", on entend une protéine étrangère à la cellule hôte qui l'exprime, c'est-à-dire d'origine différente de celle de la cellule hôte qui, selon l'invention, est une levure. La protéine peut être d'origine bactérienne, par exemple la béta-galactosidase d'Escherichia coli, ou d'origine d'eucaryotes supérieurs, par exemple d'origine humaine, telle que par exemple les lymphokines, les facteurs de la coagulation sanguine, des hormones, des antigènes vaccinaux, et, d'une manière générale, toute protéine d'intérêt thérapeutique ou industriel.

Comme cela a été indiqué, l'élément essentiel de l'invention est l'association d'une séquence de contrôle de transcription et d'un récepteur complexé à un ligand. Le choix de la première est conditionné par le choix du deuxième.

Parmi les séquences de contrôle de transcription utilisables, on peut citer en particulier les séquences habituellement fonctionnelles dans les cellules eucaryotes supérieures, c'est-à-dire des séquences naturelles d'activation de la transcription de cellules eucaryotes supérieures, telle que par exemple un HRE "pour "hormone responsive element") ou un variant ou un dérivé synthétique d'une telle séquence, et qui dans la levure remplit également cette fonction d'activation de transcription. Ces séquences sont, en général, des séquences palindromiques parfaites ou imparfaites, naturelles ou synthétiques.

Ainsi, par exemple, dans le cas où la séquence contrôle de transcription utilisée est une séquence HRE, le récepteur est complexé à l'hormone correspondante.

Comme indiqué précédemment, la séquence contrôle de transcription représente un des éléments assurant l'expression dans la levure de la protéine désirée. L'expression dans la levure peut donc être assurée par des séquences hybrides qui comprennent, outre la séquence contrôle de transcription définie dans le cadre de l'invention, des séquences correspondant à des séquences de promoteurs de levure. Parmi les promoteurs utilisables, on peut citer le promoteur inductible GAL 1 mentionné précédemment, mais aussi des promoteurs constitutifs de levure tels que le promoteur PGK.

Généralement, on conserve l'élément TATA du promoteur et on peut placer la séquence contrôle de transcription à des distances variables en amont de l'élément TATA. Il est particulièrement intéressant de pouvoir disposer d'une structure minimale pour le promoteur hybride, c'est-à-dire une structure où la distance entre l'élément TATA et la séquence contrôle de transcription est faible tout en demeurant suffisante pour assurer une inductibilité satisfaisante.

On entend donc par "récepteur" au sens de l'invention, une protéine fonctionnelle dans des cellules eucaryotes supérieures, ou des variants ou dérivés synthétiques correspondants qui présentent dans la levure les propriétés fonctionnelles du récepteur natif.

Le terme "récepteur" comprend également des protéines chimériques, c est-à-dire une structure dans laquelle les deux parties essentielles sont d'origines différentes. Par exemple, une combinaison préférée consiste à préparer un récepteur hybride dans lequel la partie qui reconnaît le ligand est d'origine eucaryote supérieure et la partie qui se fixe sur la séquence de contrôle de transcription est d'origine de levure, mais il est possible d'utiliser des séquences provenant de tout autre microorganisme, par exemple des bactéries. La séquence de contrôle de transcription comprend alors une séquence fonctionnelle dans les levures, c'est-à-dire une séquence naturelle d'activation de la transcription dans les levures. On peut citer, par exemple, l'UAS du promoteur GAL1 qui est un promoteur de levure.

L'invention a donc pour objet les différentes combinaisons fonctionnelles entre une séquence de contrôle de transcription, qu'elle soit naturelle ou dérivée, notamment par synthèse chimique, et le récepteur qui peut être un récepteur naturel, dérivé ou chimérique. La nature du ligand est à son tour déterminée par le choix du récepteur.

Parmi les récepteurs utilisables, il faut citer les récepteurs nucléaires, notamment à un stéroïde, et les autres récepteurs nucléaires, par exemple aux rétinoïdes ou aux hormones thyroïdiennes ainsi que la vitamine D3. Le récepteur à un stéroïde peut être un récepteur naturel aux hormones stéroïdes, par exemple le récepteur aux oestrogènes, le récepteur à la progestérone, le récepteur à la testostérone ou un recepteur variant ou un dérivé chimérique, fonctionnel vis-à-vis de la séquence contrôle de la transcription. Le stéroïde peut être un stéroïde naturel tel que l'oestradiol, la progestérone, la testostérone ou un analogue ou un dérivé, fonctionel dans le complexe auquel est sensible la séquence contrôle de la transcription que renferme le vecteur de l'invention.

Le récepteur aux oestrogènes peut être le récepteur naturel (noté plus loin hER) ou un variant ou un dérivé chimérique, fonctionnel en présence d'oestradiol vis-à-vis de la séquence contrôle de la transcription que renferme le vecteur de l'invention.

Plus particulièrement, l'invention a pour objet le cas où la partie essentielle du récepteur reconnaissant le ligand provient du récepteur oestrogène humain désigné par hERG qui, par rapport au récepteur désigné par hER, présente une glycine au lieu d'une valine en position 400, le ligand étant alors l'oestradiol. Suivant une caractéristique de l'invention, on pourra utiliser en tant que récepteur, la totalité du récepteur hERG.

Par rapport au récepteur oestrogène humain hER dont la séquence d'ADN complémentaire avait été publiée dans Green S. et al. (1986) (Nature, 320, 134-139), le récepteur hERG comprend donc une glycine à la place d'une valine en position 400.

On a pu constater qu'en utilisant ce récepteur au lieu du récepteur hER, on obtenait une plus grande stabilité du récepteur, en supprimant donc tout risque de dénaturation pouvant conduire à une plus faible fixation du ligand spécifique, en l'occurrence l'oestradiol. Ce phénomène est particulièrement vrai aux environs de 25°C, c'est-à-dire aux températures où est mis en oeuvre le procédé de préparation de protéines recombinantes comprenant une étape d'induction selon l'invention. Il est donc préférable d'utiliser ce récepteur qui permet de mettre une moindre quantité d'oestradiol dans le milieu de culture pour déclencher l'induction.

Selon une caractéristique supplémentaire de l'invention, l'oestradiol est ajouté à une concentration comprise entre 2 et 50 nM, de préférence de l'ordre de 10 nM.

L'invention concerne particulièrement le cas dans lequel la séquence de contrôle est la séquence -605 à -634 du gène de la vitellogénine de poulet qui est la séquence contrôle de la transcription de ce gène, sensible à l'oestradiol (notée plus loin ERE pour "estrogen responsive element"). La synthèse d'oligonucléotides, renfermant cette séquence ou des répétitions de celle-ci permet d'obtenir, selon l'invention, un vecteur d'expression comprenant cette séquence ou des répétitions de celle-ci (notée plus loin ERE1 ou ERE3).

Un des avantages de l'invention est que l'induction de l'expression peut être déclenchée de façon très simple, par ajout au milieu de culture d'un ligand adapté au récepteur. La mise en oeuvre de l'induction devient donc totalement indépendante de la nature du milieu de culture.

Le moment convenant pour l'introduction du ligand peut être facilement déterminé ; en pratique, il s'agira du moment où un haut niveau de biomasse a été atteint dans la cuve de fermentation.

L'invention concerne tout particulièrement les vecteurs dont la structure et le procédé d'obtention seront donnés plus loin à titre d'exemple dans la partie expérimentale.

Il convient de remarquer que les éléments d'expression de la protéine et les éléments assurant l'expression du récepteur peuvent se trouver sur un seul plasmide ou sur deux plasmides différents comportant chacun une origine de réplication fonctionnelle dans les levures. Mais il est également possible de prévoir l'utilisation de vecteurs assurant l'intégration de certains de ces éléments, par exemple la production du récepteur peut provenir de séquences intégrées sur un chromosome de la cellule.

C'est pourquoi la présente invention concerne également les vecteurs portant les séquences utilisables pour la transformation des cellules de levures.

L'invention s'étend aussi aux souches de levures utilisables dans le procédé selon de l'invention.

Comme souches de levures, toutes les souches utilisées habituellement pour l'obtention de protéines recombinantes peuvent être utilisées. On peut citer notamment les souches de Saccharomyces telles que S. cerevisiae. En particulier, on peut citer les souches présentant une ou plusieurs déficiences en activité protéolytique, par exemple présentant la mutation pep4. On peut citer également les souches présentant une suppression de la fonction protéolytique codée par le gène PRC1. L'utilisation de ces souches permet d'améliorer la qualité des protéines obtenues.

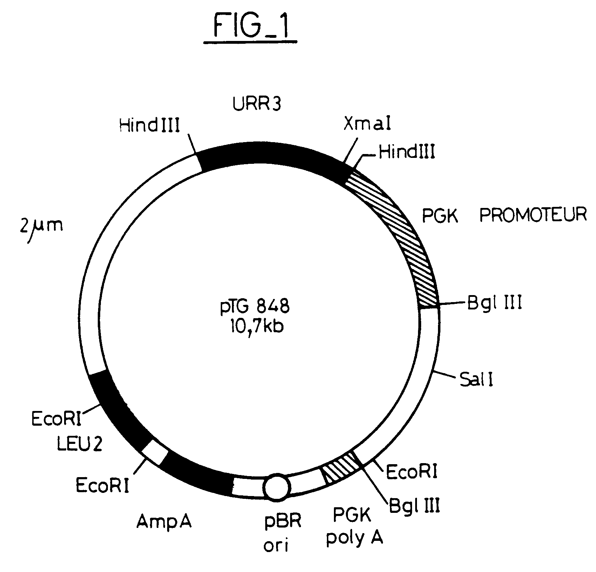

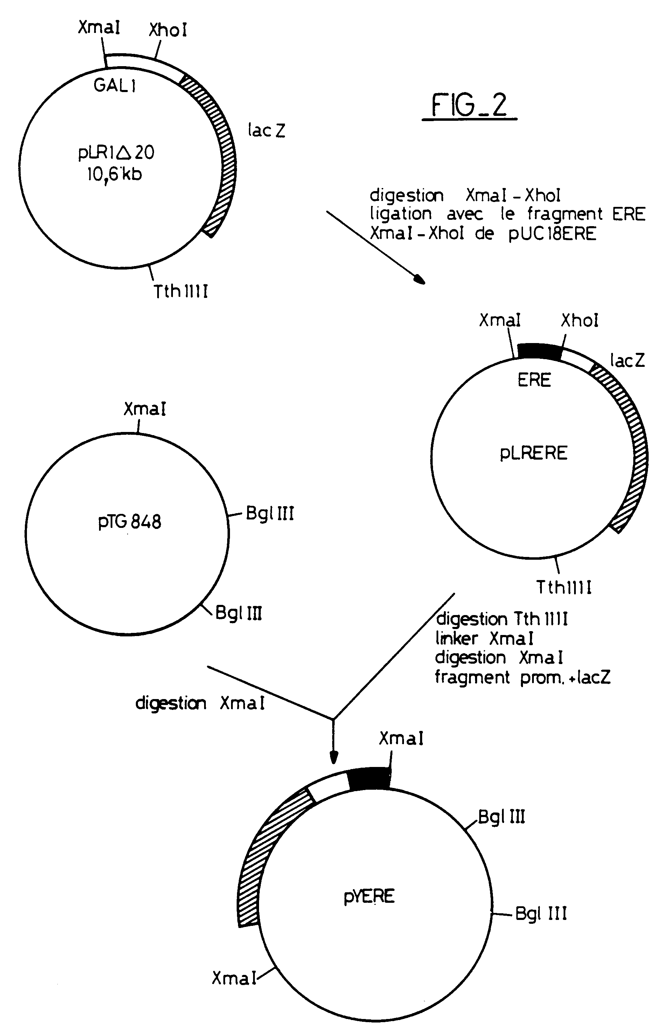

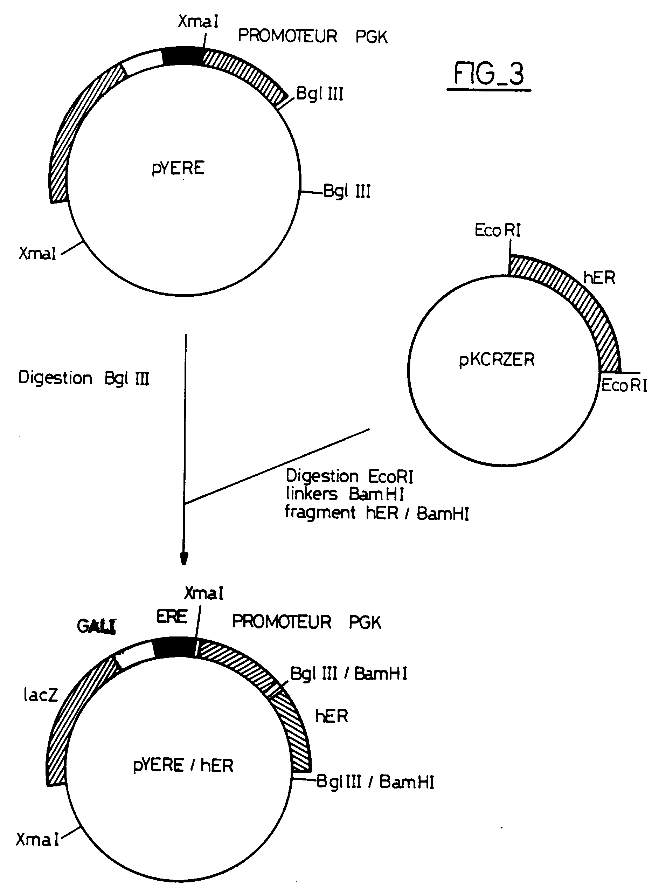

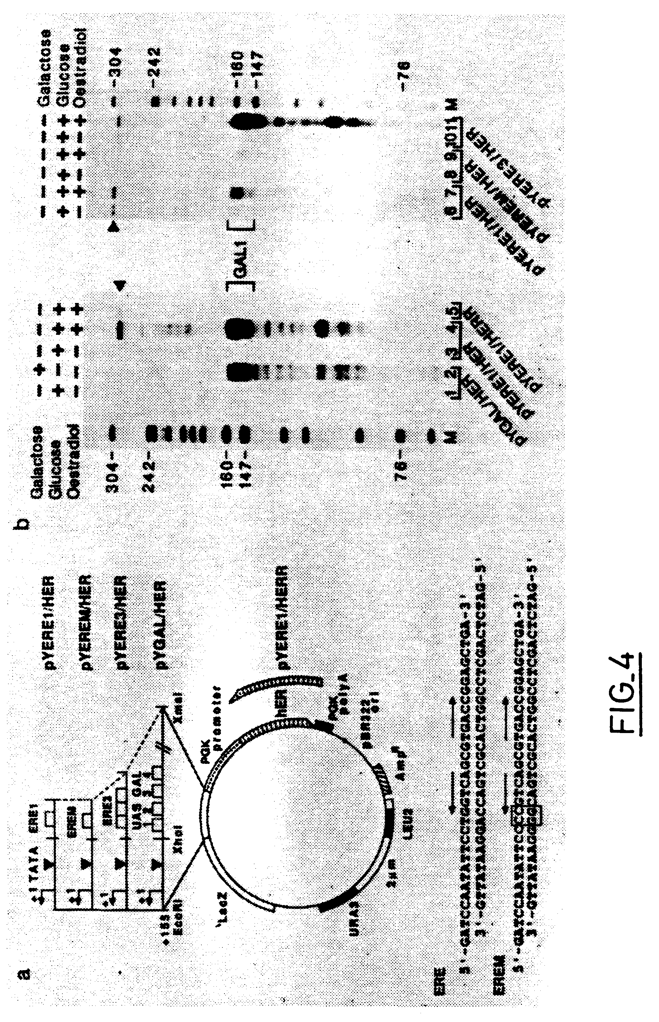

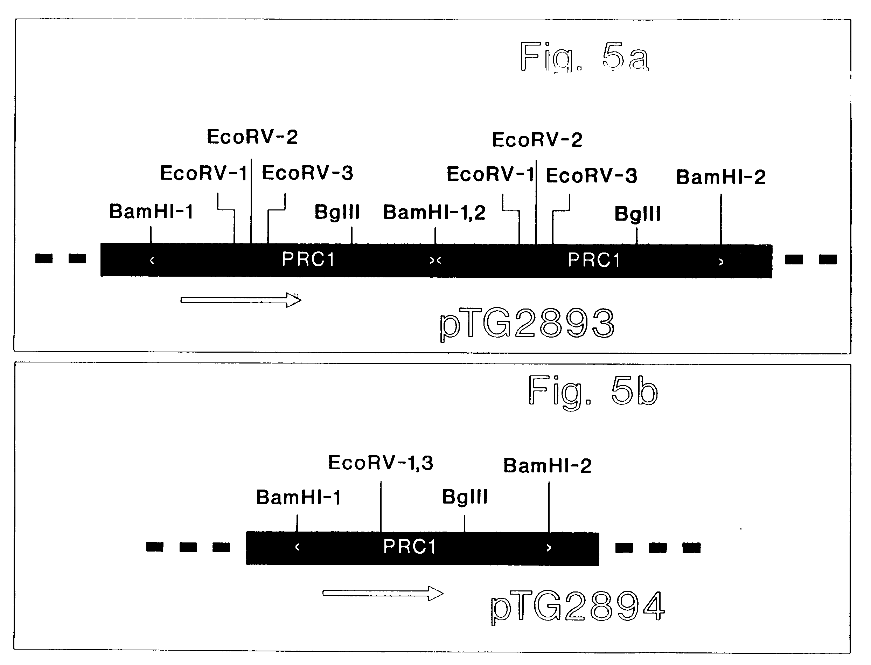

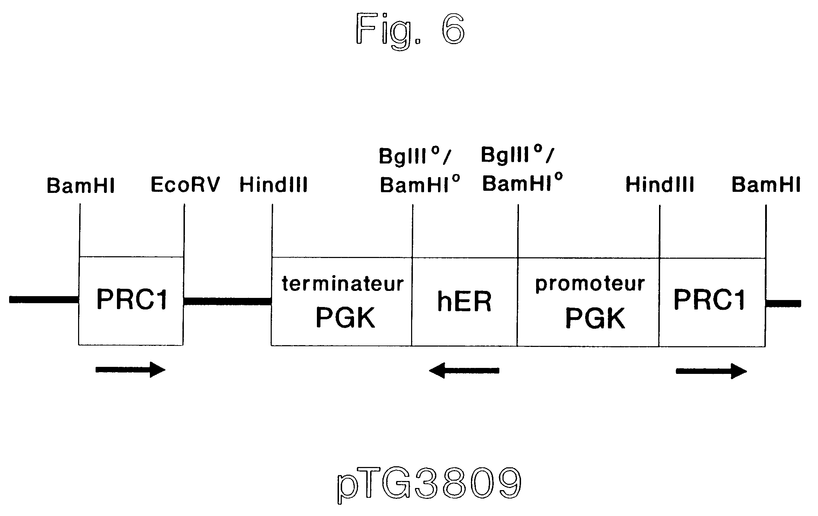

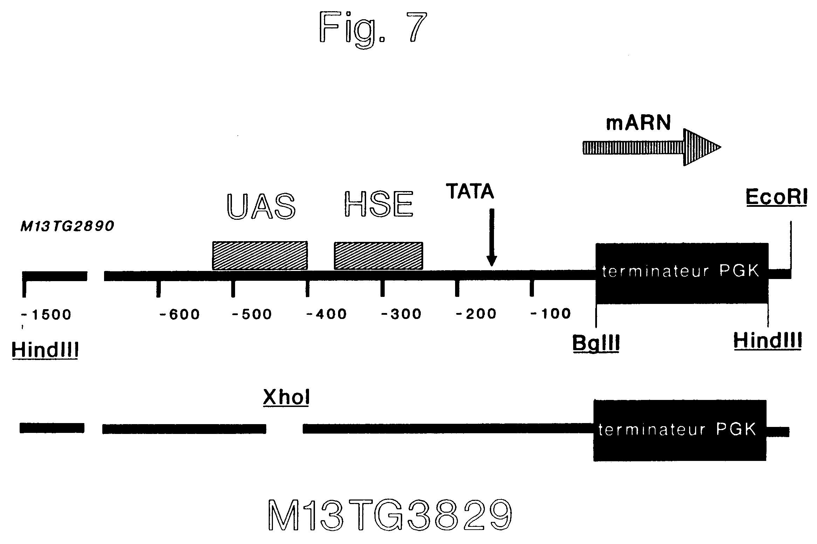

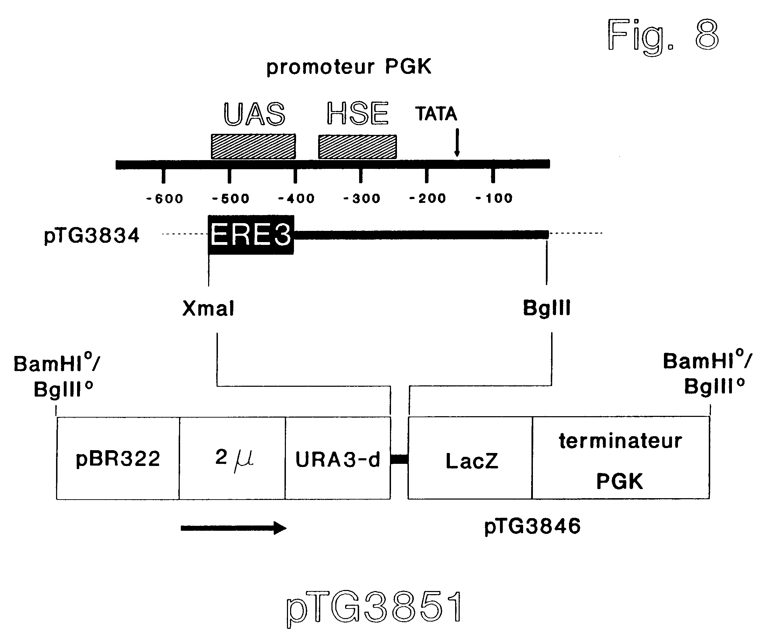

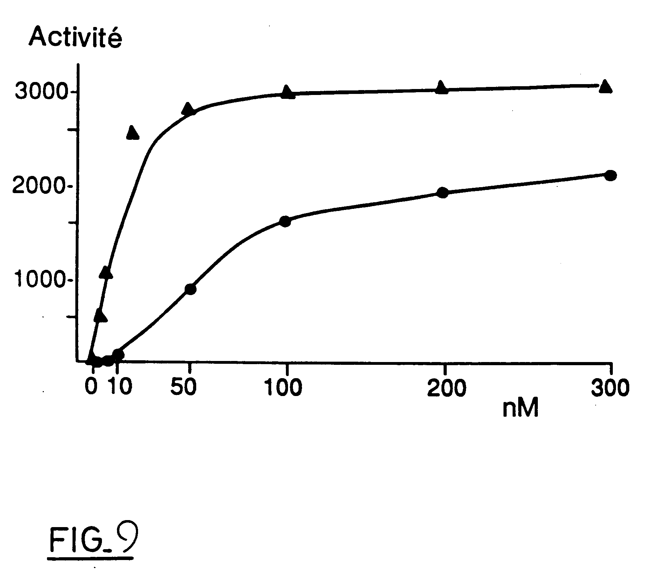

Sur les figures annexées,
. la figure 1 représente la structure de pTG848,
. la figure 2 schématise la stratégie permettant de préparer pYERE,
. la figure 3 schématise la stratégie permettant de préparer pYERE/hER,
. la figure 4a schématise les structures de pYERE1/hER, pYEREm/hER, pYERE3/hER, pYGAL/hER et montre les séquences des éléments ERE et EREm,
. la figure 4b montre, pour les ARN extraits des cellules induites ou non induites contenant les plasmides indiqués, les fragments ARN protégés de la digestion par les ribonucléases A et T1 après hybridation avec une sonde ARN Gal1 anti-sens,
. la figure 5a représente schématiquement le résultat de l'insertion du tandem d'une partie de la séquence codante de PRC1 dans pUC8 (site BamHI) pour donner pTG2893,
. la figure 5b schématise la délétion introduite dans le fragment de PRC1 par l'excision du fragment EcoRV de pTG2893 pour donner pTG2894, les flèches représentent le sens de transcription,
. la figure 6 représente la structure schématique du plasmide pTG3809, les flèches représentent le sens de la transcription,
. la figure 7 représente la structure schématique du phage M13TG2890,
. la figure 8 représente schématiquement la construction du plasmide pTG3851,
. la figure 9 met en évidence l'effet sur l'activation de la transcription dans des cellules de levures de l'ajout de quantités croissantes d'oestradiol avec comme récepteur, soit le récepteur oestrogène humain hER (cercles), soit le récepteur oestrogène humain hERG (triangles).

Les exemples suivants illustrent l'invention sans toutefois la limiter.

### EXEMPLE 1 : Construction des vecteurs pYERE/hER

### Méthodes :

Les techniques de Biologie Moléculaire utilisées pour la construction des différents plasmides sont décrites dans les manuels "Molecular cloning a laboratory manual", T. Maniatis, E.F. Fritsch et J. Sambrook, Cold Spring Harbor, New York : Cold Spring Harbor Laboratory (1982) et "Current Protocols in Molecular Biology" F.M. Ausubel, R. Brent, R.E. Kingston, D.D. Moore, J.A. Smith, J.G. Seidman et K. Sruhl, Greene Publishing Associates and Wiley Intersciences (1987). Elles comprennent notamment la digestion de l'ADN par les enzymes de restriction, la séparation des fragments ADN par électrophorèse en gel agarose ou polyacylamide, la ligation de fragments ADN par l'enzyme T4 DNA ligase, la transformation de E. coli par les plasmides recombinants, la détermination de la séquence nucléotidique par la technique de Sanger.

Les plasmides utilisés portent le gène de résistance à l'ampicilline, ce qui permet la sélection des bactéries transformées par un plasmide sur boite agar contenant de l'ampicilline (50 »g/ml).

La souche de E. coli utilisée est la souche DH5 génotype : F-, endA1, HsdR17(rk-md+), supE44, thi-1, lambda-, reca&, gyrA96, reIAI (Hanahan DNA cloning : a practical approach (Vol. 1= p. 109-135, ed. D.M. Glover, IRL Press, Oxford (1985)).

Les techniques de transformation et de croissance des souches de levure sont décrites dans "Laboratory Course Manual for Methods in Yeast Genetics", F. Sherman,G.R. Find et J.B. Hicks, Cold Spring Harbor, New York : Cold Spring Harbor Laboratory (1986).

La souche de levure utilisée est la TGY14-1a, pep4.3 genotype Mat a, ura3-251-373-328, leu2, pep4.3
le plasmide parent pTG848, décrit dans le brevet n° EP200655, est un plasmide navette E. coli/levure, qui permet l'expression dans la levure d'un gène exogène sous le contrôle du promoteur du gène phosphoglycerate kinase (PGK). Ce plasmide contient une origine de réplication pBR 322 et le gène de résistance à l'ampicilline pour la sélection dans E. coli, une origine de réplication 2 »m et les gènes ura3 et leu2 pour la sélection dans la levure, ainsi que le promoteur du gène PGK et son extrémité 3' contenant le signal de polyadénylation. Ce gène a été modifié de manière à introduire un site BglII au niveau de l'ATG initiateur, ce qui permet d'insérer la région codante d'une autre protéine entre ce site et le site BglII situé en amont du signal de polyadénylation (figure 1)

### Construction des plasmides pUC18 ERE1, pUCU18 EREm, pUC18 ERE3 :

le plasmide pUC18 (C. Yanisch-Perron, J. Vieira et J. Messing, Gene 33, 103-1190 (1986)) contient une origine de réplication pBR322, le gène de résistance à l'ampicilline et un polylinker comprenant notamment les sites XbaI et HindIII. Ce plasmide a été digéré par les enzymes de restriction XmaI et HindIII ; au plasmide ainsi digéré on ajoute alors par les techniques oligonucléotides complémentaires :
5'-CCGGGTCTAGAAGATCTA3'
3'CAGATCTTCTAGATTCGA5'
Le plasmide dérivé pUC18 BglII a alors un polylinker qui contient dans l'ordre les sites XmaI, XbaI, BglII et HindlII.

Dans ce plasmide digéré par BglII, on introduit respectivement les fragments constitués des oligonucléotides complémentaires :
- ERE1: : 5'GATCCAATATTCCTGGTCAGCGTGACCGGAGCTGA3'
3'GTTATAAGGACCAGTCGCACTGGCCTCGACTCTAG5'
- EREm: : 5'GATCCAATATTCCCCGTCAGCGTGACCGGAGCTGA3'
3'GTTATAAGGGGCAGTCGCACTGGCCTCGACTCTAG5'
ERE3 qui consiste en une répétition directe de trois fragments ERE1. ERE1 contient la séquence ERE((pour "estrogen responsive element")-605 à -634 du promoteur de la vitellogénine.
EREm est un mutant où une transversion GC dans la séquence ERE réduit considérablement son activité dans les cellules MCF-7.

### Construction des plasmides pLRERE1, pLREREm, pLRERE3 :

Le plasmide pLR1 ..20 est décrit dans l'article de R.W. West, R.R. Yocum et M. Ptashne, Molecular and Cellular Biology 4, 2 467-2 478 (1984). Ce plasmide contient un gène actif et chimérique de béta-galactosidase sous contrôle du promoteur GAl1 (figure 2).
le but de la construction est de remplacer les UAS (upstream activating sequence) du gène Gal1, situées entre les sites XmaI et XhoI de la région promoteur Gal1, par les fragments ERE1, EREm, ERE3, respectivement.
pLRERE1 : Le plasmide pUC18ERE1 est digéré par l'enzyme de restriction BglII. Le site est rempli avec la DNA polymerase I (fragment de Klenow) en présence des 4 déoxynucléotides triphosphates. Un linker XhoI (Biolabs ref. 1030) est alors ajouté aux extrémités : le plasmide est ensuite digéré par XmaI et XhoI et le fragment XmaI-XhoI contenant la séquence EREI est isolé.

Ce fragment est ensuite cloné dans le plasmide pLR1 20 entre les sites XmaI et XhoI pour donner le plasmide pLRERE1.

### pLREREm, pLRERE3 :

On construit de la même façon les plasmides pLREREm et pLRERE3 en remplaçant pUC18ERE1 par pUC18EREm et pUC18ERE3, respectivement, dans le protocole ci-dessus.

### Construction des plasmides pYERE1, pYEREm, pYERE3 :

Les plasmides pLRERE1, pLREREm, pLRERE3 possèdent à l'extrémité 3' du gène chimérique lacZ (beta-galactosidase) un site Tth111I (figure 2).

### pYERE1 :

le plasmide pLRERE1 est digéré par l'enzyme Tth111I ; le site est ensuite rempli avec la DNA Polymérase I (fragment de Klenow). Un linker XmaI (Biolabs ref. 1048) est alors ajouté aux extrémités. L'ADN est digéré par XmaI, et le fragment XmaI-XmaI contenant le gène chimérique lac Z sous contrôle du promoteur hybride (EREA-Gal1) est inséré au site unique XmaI du plasmide pTG848, pour donner le plasmide pYERE1.

### pYEREm, pYERE3 :

On construit de la même façon les plasmides pYEREm et pYERE3 en remplaçant pLRERE1 par pLREREm et pLRERE3, respectivement, dans le protocole ci-dessus.

### Construction du plasmide pYGAL :

Le plasmide pLR1 20 est digéré par l'enzyme de restriction Tth111I, le site est ensuite rempli avec l'ADN polymérase I (fragment de Klenow). Un linker XmaI (Biolabs ref. 1048) est ajouté aux extrémités, puis l'ADN est digéré par l'enzyme XmaI. Le fragment XmaI contenant le gène chimérique lacZ sous contrôle du promoteur Gal1 est inséré au site XmaI du plasmide pTG848, donnant le plasmide pYGAL.

### Construction des plasmides pYERE1/hER, pYERE1/hERR, pYEREm/hER, pYERE3/hER, pYGAL/hER :

Le but de ces constructions est de placer le cADN codant pour le récepteur oestrogène humain (hER) sous contrôle du promoteur PGK, au site BglII des plasmides pYERE1, pYEREm, pYERE3, pYGAL (fig. 3).

Le plasmide pKCR2-ER contenant le cADN du récepteur oestrogène humain (S. Green et coll. Nature 320, 134-139 (1986)) est digéré par l'enzyme de restriction EcoRI, le site est ensuite rempli par l'ADN polymérase I (fragment de Klenow). Un linker BamHI (Biolabs ref. 102) est alors ajouté aux extrémités. Après digestion par BamHI, le fragment codant pour le récepteur oestrogène humain est isole.

Il est inséré dans le vecteur pYERE1 digéré par BglII pour créer les plasmides pYERE1/hER et pYERE1/hERR, qui diffèrent par l'orientation du cADN hER relativement au promoteur PGK.

Dans pYERE1/hER, le promoteur PGK est situé en 5' du cADN hER et induit donc la transcription de l'ARNm codant pour la protéine hER.

Dans pYERE1/hERR, l'orientation est inverse et induit donc la transcription d'un ARN anti-sens de l'ARN messager hER.

pYEREm/hER, pYERE3/hER, pYGAL/hER sont construits sur le même modèle que pYERE1/hER en remplaçant pYERE1 par pYEREm, pYERE3, pYGAL, respectivement, dans la construction précédente.

Ces plasmides (fig. 4a) construits dans E. coli DH5 sont transférés dans la souche de levure TGY14.1a qui porte la mutation ura3. La présence du gène ura3 sur ces plasmides permet la complémentation, pour les levures transformées par un plasmide, qui sont donc sélectionnées sur boîte agar contenant un milieu sans uracile (préparation : 0,67 g milieu Difco n° 0919-15, 0,5 g casaminoacides, 1 g glucose, 2 g Agar, pour 100 ml).

### EXEMPLE 2 : Induction par l'oestradiol de la transcription pour les promoteurs hybrides ERE Gal1 portés par les vecteurs pYERE/hER :

Les différentes levures contenant les plasmides pYERE1/hER, pYEREm/hER, pYERE3/hER, pYERE1/hERR, pYGAL/hER, respectivement, sont cultivées dans le milieu de base pour levure (Difco n° 0919-15) à raison de 0,67 g/100 ml et 0,5 % casaminoacides contenant du glucose 1 % ou du galactose 2 % ou 100 nM oestradiol (ajouté à la culture 2 heures avant de récupérer les cellules) comme indiqué fig. 4b.

L'ARN est préparé à partir de 10⁹ cellules selon le protocole décrit dans Methods in Yeast Genetics, p 143-144.

Pour mesurer l'activité transcriptionnelle des promoteurs Gal1 ou ERE-Gal1, ces ARN sont hybridés avec une sonde ARN radiomarquée dérivee de l'extrémité 5' du gène Gal1 (brin complémentaire de l'ARN messager). Les fragments hybridés seront protégés de la digestion par les RNases A et T1. La sonde est préparée à partir du fragment EcoRI (contenant le promoteur Gal1) du plasmide pLR1 20 (R.W. West, R.R. Yocum et M. Plashne, Molecular and Cellular Biology 4, 2467-2478 (1984)) cloné dans le plasmide Bluescribe M13+ (Stratagène ref. 211201). Le plasmide résultant est ensuite linéarisé par HindIII et la sonde radiomarquée est ensuite synthétisée à partir du promoteur du phage T7 avec le Kit Stratagène n° 211604. La taille de la sonde est de l'ordre de 1000 nucléotides.

Il existe plusieurs sites d'initiation de la transcription dans le promoteur Gal1, la sonde est complémentaire de l'ARN de Gal1 sur une longueur de 154 à 165 nucléotides.

La sonde est incubée avec 20 »g d'ARN et le mélange contenant les hybrides éventuels est digéré avec les ribonucléases A et T1 selon le protocole décrit dans Current Protocols in Molecular Biology.

Les fragments non digérés sont ensuite identifiés par électrophorèse en gel polyacrilamide-urée et autoradiographie (fig. 4b).

Pour les constructions exprimant le récepteur hER, on observe, en présence d'oestradiol, l'induction d'un ARN messager spécifique du promoteur hybride ERE-Gal1 ; les sites d'initiation de la transcription sont identiques à ceux du promoteur Gal1 (pYGAL) induit par le galactose : fragments protégés de même taille.

L'induction de la transcription à partir du promoteur hybride ERE-Gal1 est strictement dépendante de la présence d'oestradiol. L'induction par l'oestradiol nécessite la présence du récepteur hER (la construction pYERE/hERR est inactive). Elle nécessite également la présence d'un élément ERE (pour la construction pYGAL/hER il n'y a pas d'induction par l'oestradiol.

La quantité d'ARN Gal1 détectée pour les cellules contenant pYERE3/hER est environ 10 fois plus élevée que pour celles contenant pYERE1/hER : la répétition en tandem des éléments ERE (pYERE3 contient un trimère ERE, pYERE1 un monomère) paraît donc avoir un effet de synergie sur l'activation de la transcription.

### EXEMPLE 3 : Expression inductible par l'oestradiol de la béta-galactosidase dans des levures contenant les vecteurs pYERE/hER

L'activité béta-galactosidase est mesurée selon le protocole de West et coll. (1984). Le tableau 1 ci-après montre l'activité béta-galactosidase des souches de levure portant respectivement les plasmides pYERE1/hER, pYERE3/hER, pYEREm/hER, pYERE1/hERR, pYGAL/hER en présence ou absence du glucose, galactose ou oestradiol, comme indiqué dans le tableau.

Le niveau d'activité constitutive des promoteurs est très bas et n'est pas affecté par la présence de la protéine hER (pYGAL et pYGAL/hER ou pYERE1/hER et pYERE1/hERR, donnent le même taux d'activité). Par contre, l'addition d'oestradiol entraîne une forte induction des promoteurs hybrides de pYERE1/hER et pYERE3/hER.

Le fait que les promoteurs de pYEREm/hER et pYGAL/hER ne soient pas sensibles à l'induction par l'oestradiol montre la nécessité d'un élément ERE fonctionnel, dans le promoteur contrôlant l'expression de lacZ, tandis que l'absence d'induction pour pYERE1/hERR montre que la présence du récepteur hER est également nécessaire pour observer l'induction par l'oestradiol.

Le niveau d'expression atteint est comparable à celui obtenu à partir du promoteur Gal1 (cas de pYGAL/hER) en présence de galactose.

La présence de trois éléments ERE en tandem (cas de pYERE3/hER) donne une activité deux fois supérieure au cas où le promoteur contient un seul élément ERE (pYERE1/hER).

**Tableau I**

| Induction en présence d'hormone de l'activité béta-galactosidase par le yhER | | | | |
|---|---|---|---|---|
| PLASMIDE | MILIEU | | | ACTIVITE BETA-GALACTOSIDASE (unité) |
| | Glucose | Galactose | Oestradiol | |
| pYERE1/hER | + | - | - | 60 |
| pYERE1/hER | + | - | + | 1 060 |
| pYERE1/hERR | + | - | - | 60 |
| pYERE1/hERR | + | - | + | 60 |
| pYERE3/hER | + | - | - | 60 |
| pYERE3/hER | + | - | + | 2 060 |
| pYEREM/hER | + | - | - | 60 |
| pYEREM/hER | + | - | + | 260 |
| pYGAL/hER | + | - | - | 180 |
| pYGAL/hER | + | - | + | 180 |
| pYGAL | + | - | - | 180 |
| pYGAL | - | + | - | 2 300 |

Les levures transformées par les plasmides indiqués sont mises en culture en présence de 1 % de glucose ou 2 % de galactose, comme mentionné dans le tableau ; pour l'induction par l'oestradiol, on ajoute 100 nM d'hormone 2 heures avant la récolte des cellules.

Les tests d'activité béta-galactosidase sont effectués de manière classique (West R.W. jr., Yocum R.R. et Ptashne M., Mol. Cell. Biol., 4, 2467-2478 (1984).

### EXEMPLE 4 : Intégration d'un ADN complémentaire codant pour hER dans le génome d'une souche de levure

### Préparation du vecteur d'expression portant une cassette d'expression de hER insérée dans le gène PRC1 (pTG3809)

La cassette d'expression de hER est insérée dans un fragment du gène PRC1 afin d'obtenir après transformation d'une souche de levure par pTG3809 une souche de levure présentant une suppression de la fonction protéolytique codée par le gène PRC1.

On prépare tout d'abord une cassette d'expression de hER (M13TG2896) de la façon suivante :
- le promoteur de PGK de levure (fragment HindIII-BglII) et son terminateur (fragment HINDIII-BglII) sont introduits dans le phage M13TG130 (Kieny, M.P. et al. (1983) Gene 26, p91-99) linéarisé par digestion HindIII pour donner le phage M13TG2890,
- le fragment BamHI dérivé de pKCR2-hER portant le récepteur oestrogène humain est introduit dans M13TG2890 linéarisé par digestion BglII pour donner une cassette d'expression de hER appelée M13TG2896.

Cette cassette d'expression de hER est ensuite insérée dans le gène PRC1 pour donner le plasmide pTG3809 permettant son intégration au génome de la levure.

La séquence du gène PRC1 a été publiée par Valls, L.A. et al. (1987) (Cell 48, p887-897). Deux oligonucléotides sont construits à partir de cette séquence. Le premier est complémentaire de la région 5' du gène et sa séquence est la suivante :
5' AAG AAA GAC TGG GAC TTT GTG 3'
Le second est complémentaire de la région 3' du gène et sa séquence est la suivante :
5' GAT TGG ATG AAG CCT TAC CAC 3'
A partir d'une banque génomique de levure (fragments d'ADN chromosomique partiellement digéré par Sau3A insérés dans le site BamHI de pFL1 (Parent, S.A. et al. (1985) Yeast 1 p83-138) et par hybridation avec ces deux oligonucléotides on sélectionne un clone de E. coli contenant le gène PRC1 inséré dans pFL1 et appelé pTG2863.

Le fragment BamHI de 1,1kb de pTG2863 portant une partie de la séquence codante du gène PRC1 est introduit dans le site BamHI du plasmide pUC8. Cette insertion s'effectue en tandem et on obtient le plasmide pTG2893 (figure 5a).

Les coordonnées de la première base des sites de restriction par rapport à l'adénine de l'ATG de la séquence codante de PRC1 sont les suivantes : BamHI-1 : 482; EcoRV-1 : 1102; EcoRV-2 : 1153; EcoRV-3 : 1234 et BamHI-2 : 1574.

Une délétion dans la séquence codante de PRC1 est effectuée par digestion EcoRV de pTG2893 pour donner pTG2894 (figure 5b).

La cassette d'expression de hER est introduite dans le fragment interne délété du gène PRC1 de pTG2894 de la façon suivante : la cassette d'expression (M13TG2896) est digérée par BamHI, les extrémités sont remplies à la polymérase de Klenow et après clivage par EcoRV un fragment est isolé. Ce fragment porte la cassette entière, il est introduit dans le site EcoRV de pTG2894 pour donner le plasmide pTG3809 (figure 6) qui porte entre deux fragments de PRC1 :
- plusieurs sites de reconnaissance pour les enzymes de restriction SphI, XbaI, KpnI provenant de M13TG130,
- le terminateur de PGK de la levure,
- l'ADN complémentaire de hER,
- le promoteur de PGK de la levure.

### Transformation d'une souche de levure par le plasmide portant la cassette d'expression hER insérée dans un fragment du gène PRC1 délété (pTG3809)

La souche de levure transformée est une souche de S. cerevisiae TGY2sp13b (MATa; ura3-251, -373, -328; leu2-3, -112).

Le plasmide pTG3809 est clivé par BamHI. Les séquences d'ADN des extrémités du fragment ainsi isolé sont homologues à celles de la séquence du gène PRC1. Ces séquences vont permettre l'intégration du fragment dans la partie génomique du gène PRC1 de la souche de levure. Cette insertion entraîne la destruction du gène PRC1 génomique par déletion.

Comme le fragment ne porte pas de marqueur de sélection, le plasmide pLRERE3 est utilisé comme vecteur de cotransformation. Il permet la sélection de prototrophes Ura⁺. Parmi les prototrophes Ura⁺ les clones qui ont intégré la cassette d'expression hER portent une mutation dans le gène PRC1 qui entraîne une perte de l'activité de la carboxypeptidase yscY. Ces mutants sont détectés par un test colorimetrique (Jones, E.W. (1977) Genetics 85 p23-33). La structure du locus PRC1 modifié est vérifiée par la méthode de Southern.

Cette souche de S. cerevisiae ayant intégré la cassette d'expression de hER dans le gène PRC1 délété est appelée TGY2sp13b prc1-d::hER.

### Construction d'un plasmide portant la séquence ERE3 et un fragment du promoteur du gène PGK (pTG3851)

Un site XhoI est introduit dans le vecteur M13TG2890 à la jonction 5', 3' des extrémités de la délétion effectuée par mutagénèse dirigée en utilisant l'oligonucléotide suivant : 
-506 . . -401
5' AATTACCGTCGCTCG AGCGACGGCTCACAG 3'
XhoI
pour donner le vecteur M13TG3829 (figure 7). les positions indiquées sont déterminées par rapport à l'ATG du gène PGK. Par la suite la forme délétée du promoteur de PGK sera notée PGK-dp401. La conformité de la séquence est ensuite vérifiée par séquençage.

Ce vecteur porte la séquence TATA et le site d'initiation de la transcription, la seconde séquence activatrice en aval notée HSE, de -256 à -377 (Piper, P.W. et al. (1988) Nucleic Acids Res. 16 p1333) mais ne porte plus l'UAS de -402 à -479. Le site XhoI permet la ligation avec la séquence ERE3 et le site BglII l'introduction d'un gène qui permet de détecter une activité par test enzymatique.

La souche hôte des phages M13 est la souche E. coli JM103 (delta(lac, pro), thi, strA, supE, endA, sbcB, hsdR, F'traD36, proAB, lacIQ, lacZdeltaM15).

Le plasmide pLRERE3 est digéré par XmaI et EcoRI. Le fragment d'ADN contenant les séquences ERE3 est purifié sur gel d'agarose puis inséré dans les sites XmaI-EcoRI du plasmide pTZ18R (Pharmacia, LKB Biotechnology) pour donner le plasmide pTG3833. La souche hôte des plasmides est la souche E. coli 1106 (supE, hsdR, hsdM, met, supF).

Le phage M13TG3829 (figure 7) est traité par XhoI et EcoRI puis est inséré au niveau du site XhoI-EcoRI du plasmide pTG3833 pour donner pTG3834.

Le gène lacZ est sorti de pCH110 (Hall, C.V. et al. (1983) J. Mol. Appl. Gen. 2 p101-109) par digestion HindIII-BamHI et cloné dans pPolyII (Lathe, R. et al. (1987) Gene 57 p193-201) pour donner pTG1174. Le plasmide pTG2145 correspond au plasmide pTG1174 auquel les sites HindIII, PstI et SalI ont éte supprimés. Il contient dans un fragment BglII-BamHI la fusion de gène de E. coli suivante : gpt::trpS::lacZ.

Les séquences au voisinage de l'ATG sont modifiées de manière à permettre une traduction correcte dans la levure de la façon suivante :
- pTG2145 est digéré par BglII et KpnI et le fragment de 200 paires de bases contenant l'ATG purifié sur gel acrylamide.
- ce fragment est inséré au niveau des sites BamHI-KpnI du phage M13TG131 (Kieny, M.P. et al. (1983) Gene 26 p91-99) pour donner M13TG3838;
- la séquence "AGC" correspondant au second codon pour une serine de M13TG3838 est échangée par la séquence "TCT", les cytosines en position -3 et -1 par rapport à l'ATG sont remplacées par des adénines et un site BglII est généré en 5' de l'ATG pour permettre une fusion avec des gènes à insérer en aval du promoteur hybride, en effectuant une mutagénèse dirigée à l'aide de l'oligonucléotide suivant :
   5' CGATGTATTTTTCAGACATTTTAAGATCTCCAGCCTGTTT 3'
   pour donner le phage M13TG3842. Ci-dessous, les bases modifiées lors de cette mutagénèse sont soulignées :
- GACACTTCAC**ATG**AGC- (M13TG3838)
- AGATCTTAAA**ATG**TCT- (M13TG3842)
Le fragment BglII-KpnI de 130 paires de bases de M13TG3842 est introduit dans pTG2145 pour y remplacer le fragment BglII-KpnI de 200 paires de bases. On obtient alors le plasmide pTG3843.

Le plasmide pTG848 (figure 1) est digéré par BglII puis religué pour donner pTG2886. Le grand fragment HindIII-EcoRI de pTG2886 est ligué en présence de ligase T4 au fragment HindIII-EcoRI de 2,1 kb du plasmide pFL1 (parent S.A. et al. (1985) Yeast 1 p83-138) qui porte une séquence du plasmide 2» de S. cerevsiae pour donner le plasmide pTG2886 LEU2-d, URA3-d. Le fragment HindIII de 0,9 kb du plasmide pTG2800 décrit dans la publication européenne de brevet EP-A-0 258 501 portant le gène URA3-d est alors inséré dans le site HindIII de ce plasmide pour donner pTG2886 URA3-d, delta LEU2-d. Le fragment SmaI-BglII de M13TG131 (Kieny, M.P. et al. (1983) Gene 26 p91-99) qui possède plusieurs sites de restriction est ensuite introduit dans ce plasmide pour donner pTG3828.

Le plasmide pTG3843 est digéré par BglII et BamHI, le fragment portant le gène de fusion lacZ est purifié sur gel d'agarose. Ce fragment est ensuite inséré au niveau du site BglII de pTG3846 (figure 8).

Le plasmide pTG3834 est digéré par XmaI et BglII et le fragment est introduit dans le site XmaI et BglII de pTG3846 pour donner le plasmide pTG3851 (figure 8).

### Expression inductible de lacZ

La souche de S. cerevisiae TGY2sp13b prc1-d::hER est curée de son plasmide de cotransformation pLRERE3 puis transformée par le plasmide pTG3851 par la méthode de l'acétate de lithium (Ito, H. et al. (1983) J. Bacteriol. 153 p163-168) et les prototrophes Ura⁺ sont sélectionnés. Ils sont ensuite mis en culture en erlenmeyer à 30°C sur un milieu sélectif (0,7 % de bases azotées pour levures sans acide aminé (Yeast Nitrogen Bases), 0,5 % de casamino acides, 2 % de glucose et si nécessaire 2 % d'agar). Ces cultures sont effectuées avec ou sans oestradiol. Pour l'induction par l'oestradiol, on ajoute 100 nM d'hormone 2 heures avant la récolte des cellules. Les cellules sont récoltées, lavées et broyées en présence d'inhibiteurs de protéases puis les débris cellulaires sont précipités par centrifugation (10 000 tours/min). La concentration en protéines est déterminée dans le surnageant par la méthode décrite par Bradford (1976) (Anal. Biochem. 72 p248) puis l'activité béta-galactosidase par la même méthode de dosage que celle décrite dans l'exemple 3. L'activité de la beta-galactosidase est aussi déterminée par la méthode utilisée dans l'exemple 3, le dosage est effectué sur les cellules perméabilisées.

Le tableau 2 présente les activités basale (en l'absence d'oestradiol, [- (Oest)]) et induite (en présence d'oestradiol [+ (Oest)]) de la béta-galactosidase sous le contrôle des promoteurs hybrides ERE3::PGK-dp. Cette activité est exprimée en nmol d'ONPG (O-nitrophényl-béta-D-glucopyranoside) hydrolysées par minute et par mg de protéine à 30°C ; les valeurs entre parenthèses sont directement comparables à celles présentées dans le tabelau 1, exprimées en "unité". L'inductibilité correspond au rapport activité spécifique sans oestradiol/activité spécifique avec oestradiol.

**Tableau 2**

| Vecteur:Promoteur PGK | Activité spécifique | | Inductibilité |
|---|---|---|---|
| | - (Oest) | + (Oest) | |
| pTG3851:PGK-dp401 | 20 (30) | 460 (770) | 23 |

### EXEMPLE 5 : Préparation d'un vecteur d'expression de la béta-galactosidase dans la levure qui porte également une séquence codant pour le récepteur hERG (PYERE1/hERG)

On part du plasmide pYERE1/hER. A l'aide de l'oligonucléotide suivant :
5'-GGAGCACCCAGGGAAGCTACTGT-3'
on réalise par mutagénèse localisée une mutation dans le codon GTG codant pour la valine dans l'ADN complémentaire codant pour le récepteur oestrogène humain (hER) de la façon suivante : l'ADN complémentaire codant pour le récepteur hER décrit par Green et al. (1986) (Nature 320, p134-139) est sous-cloné dans le site EcoRI du vecteur d'expression eucaryote pSG1 décrit par Green et al. (1988) (Nucl. Acids Res. 16, p369). On prépare alors de l'ADN simple brin et on réalise par mutagénèse localisée une mutation ponctuelle dans le codon GTG codant pour la valine en position 400 de façon à obtenir un codon GGG codant pour la glycine à l'aide de l'oligonucléotide défini ci-dessus (le changement de nucléotide est souligné). Les séquences d'ADN sont vérifiées. On construit alors le vecteur pYERE1/hERG en insérant l'ADN complémentaire hERG dans le vecteur pYERE1/hER.

### EXEMPLE 6 : Expression inductible par l'oestradiol de la béta-galactosidase dans des levures contenant le vecteur pYER1/hERG

Les conditions de transformation et de culture des cellules sont identiques à celles décrites précédemment. Lorsque la densité optique atteint 600, on ajoute 10 nM d'oestradiol. La figure 9 montre que 50 % de l'activité maximale de la béta-galactosidase est obtenu pour une concentration en oestradiol de 5 nM avec pYERE1/hERG (triangles) alors qu'avec pYERE1/hER (cercles) une concentration d'environ 50 nM est nécessaire.

Par ailleurs, on a pu constater que la mutation qui remplace val-400 par gly-400 dans le domaine de liaison à l'oestradiol stabilise la structure du récepteur et augmente son affinité pour l'oestradiol à 25°C.

La souche Saccharomyces TGY 14-1a pYERE3/hER a été déposée à la Collection Nationale de Cultures de Microorganismes (CNCM) de l'Institut Pasteur - 25 rue du Docteur-Roux, 75724 Paris Cédex 15 (France) - le 17 juin 1988 sous le n° I-770.

## Revendications

1. Procédé de préparation d'une protéine par des levures selon lequel :
- on cultive des cellules de levure qui contiennent :
. une séquence d'ADN codant pour ladite protéine sous le contrôle d'éléments assurant son expression dans les levures, lesdits éléments comprenant une séquence de contrôle de transcription d'origine eucaryote supérieure comprenant une séquence activatrice naturelle "HRE"", un variant ou un dérivé de celle-ci, inductible par un complexe formé par un récepteur et un ligand,
. une séquence d'ADN fonctionnelle dans la levure codant pour ledit récepteur qui est un récepteur nucléaire naturel choisi parmi les récepteurs à un stéroïde, les récepteurs aux rétinoïdes, aux hormones thyroïdiennes et à la vitamine D3, un récepteur variant ou chimérique de celui-ci, le récepteur comprenant deux parties essentielles dont l'une reconnait le ligand de façon à former un complexe avec ledit ligand et l'autre se fixe sur ladite séquence contrôle de transcription, et le ligand n'est pas nécessaire à la culture des cellules mais est susceptible de pénétrer dans lesdites cellules lorsqu'il est ajouté au milieu de culture,
- on ajoute ledit ligand dans le milieu de culture à un moment convenant pour l'induction,
- on récupère la protéine synthétisée.

2. Procédé selon la revendication 1, caractérisé en ce que la séquence contrôle de transcription "HRE" est une séquence ERE.

3. Procédé selon l'une des revendications 1 ou 2, caractérisé en ce que la séquence de contrôle de transcription est une séquence palindromique parfait ou imparfaite, naturelle ou synthétique.

4. Procédé selon la revendication 3, caractérisé en ce que ladite séquence palindromique est répétée plusieurs fois.

5. Procédé selon l'une des revendications 1 à 4, caractérisé en ce que le récepteur est un récepteur nucléaire et le ligand est le composé spécifique correspondant.

6. Procédé selon la revendication 5, caractérisé en ce que ledit récepteur est le récepteur de l'oestrogène, ledit ligand est un oestrogène et la partie essentielle reconnaissant le ligand provient du récepteur hER.

7. Procédé selon la revendication 5, caractérisé en ce que la partie essentielle du récepteur reconnaissant le ligand provient du récepteur oestrogène humain désigné par hERG, le ligand étant un oestrogène.

8. Procédé selon la revendication 7, caractérisé en ce que le récepteur est le récepteur hERG et le ligand est l'oestradiol.

9. Procédé selon l'une des revendications 7, ou 8, caractérisé en ce que l'oestradiol est ajouté à une concentration comprise entre 2 et 50 nM, de préférence de l'ordre de 10 nM.

10. Procédé selon l'une des revendications 1 à 9, caractérisé en ce que, dans la levure, la séquence d'ADN codant pour ladite protéine sous le contrôle d'éléments assurant son expression dans les levures et/ou la séquence d'ADN codant pour ledit récepteur, se trouvent sur un plasmide comportant une origine de réplication fonctionnelle dans la levure.

11. Procédé selon la revendication 10, caractérisé en ce que les deux séquences d'ADN se trouvent sur le même plasmide.

12. Procédé selon l'une des revendications 1 à 11, caractérisé en ce que la séquence d'ADN fonctionnelle dans la levure codant pour ledit récepteur est portée par un chromosome de la levure.

13. Procédé selon l'une des revendications 1 à 12, caractérisé en ce qu'on utilise une souche de levure déficiente en protéases.

14. Procédé selon l'une des revendications 1 à 13, caractérisé en ce que le ligand est ajouté à la fin de l'étape de croissance cellulaire.

15. Vecteur d'expression inductible d'une protéine déterminée dans des cellules de levures transformée par ledit vecteur, caractérisé en ce qu'il comprend :
- une séquence d'ADN codant pour ladite protéine sous le contrôle d'éléments assurant son expression dans les levures, lesdits éléments comprenant :
- une séquence de contrôle de la transcription d'origine eucaryote supérieure comprenant une séquence activatrice naturelle "HRE", un variant ou un dérivé de celle-ci, inductible par un complexe formé par un récepteur et son ligand,
lesdites cellules de levures renfermant une séquence d'ADN fonctionnelle dans la levure codant pour ledit récepteur, qui est un récepteur nucléaire naturel choisi parmi les récepteurs à un stéroïde, les récepteurs aux rétinoïdes, aux hormones thyroïdiennes et à la vitamine D3, un récepteur variant ou chimérique de celui-ci, pour la mise en oeuvre du procédé selon l'une des revendications 1 à 14.

16. Vecteur selon la revendication 15, caractérisé en ce que la séquence d'ADN codant pour ledit récepteur est portée par le vecteur d'expression inductible.

17. Vecteur d'expression selon l'une des revendications 15 ou 16, caractérisé en ce que la séquence contrôle de la transcription d'origine eucaryote supérieure est sensible au complexe formé par un récepteur à un stéroïde et le stéroïde correspondant.

18. Vecteur d'expression selon l'une des revendications 15 à 17, caractérisé en ce que la séquence contrôle de la transcription d'origine eucaryote supérieure est sensible au complexe formé par le récepteur à l'oestradiol et l'oestradiol.

19. Vecteur d'expression selon l'une des revendications 15 à 18, caractérisé en ce que la séquence contrôle de la transcription d'origine eucaryote supérieure comprend la séquence, ou des répétitions de celle-ci, de -605 à -634 du gène de la vitellogénine de poulet.

20. Vecteur d'expression selon la revendication 19, constitué par le plasmide pYERE3/hER déposé à la CNCM sous le numéro I-770.

21. Les souches de levures transformées par le vecteur d'expression selon l'une des revendications 15 à 20.

## Claims

1. Method for the preparation of a protein by yeasts, according to which:
- yeast cells which contain the following are cultured:
· a DNA sequence coding for the said protein under the control of elements providing for its expression in yeasts, the said elements comprising a transcription control sequence of higher eukaryotic origin comprising a natural "HRE" activator sequence, a variant or a derivative of this sequence, which is inducible by a complex formed by a receptor and a ligand,
· a DNA sequence which is functional in yeast, coding for the said receptor, which is a natural nuclear receptor chosen from receptors for a steroid, receptors for retinoids, for thyroid hormones and for vitamin D3 and a variant or chimeric receptor of a natural receptor, the receptor comprising two essential portions, one of which recognizes the ligand so as to form a complex with the said ligand and the other binds to the said transcription control sequence, and the ligand is not necessary for the culturing of the cells but is capable of entering the said cells when added to the culture medium,
- the said ligand is added to the culture medium at an appropriate time point for induction,
- the synthesized protein is recovered.

2. Method according to Claim 1, characterized in that the "HRE" transcription control sequence is an ERE sequence.

3. Method according to Claim 1 or 2, characterized in that the transcription control sequence is a natural or synthetic, perfect or imperfect palindromic sequence.

4. Method according to Claim 3, characterized in that the said palindromic sequence is repeated several times.

5. Method according to one of Claims 1 to 4, characterized in that the receptor is a nuclear receptor and the ligand is the corresponding specific compound.

6. Method according to Claim 5, characterized in that the said receptor is the oestrogen receptor, the said ligand is an oestrogen and the essential portion recognizing the ligand originates from the hER receptor.

7. Method according to Claim 5, characterized in that the essential portion of the receptor recognizing the ligand originates from the human oestrogen receptor designated hERG, the ligand being an oestrogen.

8. Method according to Claim 7, characterized in that the receptor is the hERG receptor and the ligand is oestradiol.

9. Method according to one of Claims 7 and 8, characterized in that oestradiol is added at a concentration of between 2 and 50 nM, and preferably of the order of 10 nM.

10. Method according to one of Claims 1 to 9, characterized in that, in the yeast, the DNA sequence coding for the said protein under the control of elements providing for its expression in yeasts and/or the DNA sequence coding for the said receptor occur on a plasmid containing an origin of replication which is functional in yeast.

11. Method according to Claim 10, characterized in that both DNA sequences occur on the same plasmid.

12. Method according to one of Claims 1 to 11, characterized in that the DNA sequence which is functional in yeast coding for the said receptor is carried by a chromosome of the yeast.

13. Method according to one of Claims 1 to 12, characterized in that a yeast strain deficient in proteases is used.

14. Method according to one of Claims 1 to 13, characterized in that the ligand is added at the end of the cellular growth stage.

15. Vector for the inducible expression of a particular protein in yeast cells transformed with the said vector, characterized in that it comprises:
- a DNA sequence coding for the said protein under the control of elements providing for its expression in yeasts, the said elements comprising:
- a transcription control sequence of higher eukaryotic origin comprising a natural "HRE" activator sequence, a variant or a derivative of this sequence, which is inducible by a complex formed by a receptor and its ligand,
the said yeast cells containing a DNA sequence which is functional in yeast, coding for the said receptor, which is a natural nuclear receptor chosen from receptors for a steroid, receptors for retinoids, for thyroid hormones and vitamin D3 and a variant or chimeric receptor of a natural receptor, for carrying out the method according to one of Claims 1 to 14.

16. Vector according to Claim 15, characterized in that the DNA sequence coding for the said receptor is carried by the vector for inducible expression.

17. Expression vector according to one of Claims 15 and 16, characterized in that the transcription control sequence of higher eukaryotic origin is sensitive to the complex formed by a receptor for a steroid and the corresponding steroid.

18. Expression vector according to one of Claims 15 to 17, characterized in that the transcription control sequence of higher eukaryotic origin is sensitive to the complex formed by the oestradiol receptor and oestradiol.

19. Expression vector according to one of Claims 15 to 18, characterized in that the transcription control sequence of higher eukaryotic origin comprises the sequence, or repetitions of the latter, from -605 to -634 of the chicken vitellogenin gene.

20. Expression vector according to Claim 19, consisting of plasmid pYERE3/hER deposited with the CNCM under number I-770.

21. Yeast strains transformed with the expression vector according to one of Claims 15 to 20.

## Patentansprüche

1. Verfahren zur Herstellung eines Proteins durch Hefen, bei dem man
- Hefezellen kultiviert, die enthalten:
· eine DNA-Sequenz, die für das genannte Protein unter der Kontrolle von Elementen codiert, die seine Expression in den Hefen gewährleisten, wobei die genannten Elemente, die eine Kontroll-Sequenz der Transcription umfassen, vom Ursprung höherer Eucaryoten sind, umfassend eine natürliche Aktivator-Sequenz "HRE", eine Variante oder ein Derivat von dieser, induzierbar durch einen von einem Rezeptor und einem Liganden gebildeten Komplex,
· eine funktionelle DNA-Sequenz in der Hefe, die für den genannten Rezeptor codiert, der ein natürlicher nuklearer Rezeptor ist, ausgewählt unter den Rezeptoren für ein Steroid, den Rezeptoren für Retinoide, für Thyroid-Hormone und für Vitamin D3, einer Rezeptor-Variante oder einer Rezeptor-Chimäre, wobei der Rezeptor zwei essentielle Teile umfaßt, von denen einer den Liganden erkennt, so daß ein Komplex mit dem genannten Liganden gebildet wird, und der andere sich an der genannten Kontroll-Sequenz der Transcription fixiert, und der Ligand bei der Kultur der Zellen nicht erforderlich, aber befähigt ist, in die genannten Zellen einzudringen, wenn er dem Kulturmilieu zugegeben wird,
- den genannten Liganden dem Kulturmilieu in einem Moment zusetzt, der für die Induktion geeignet ist,
- das synthetisierte Protein gewinnt.

2. Verfahren nach Anspruch 1, dadurch gekennzeichnet, daß die Kontroll-Sequenz der Transcription "HRE" eine Sequenz ERE ist.

3. Verfahren nach einem der Ansprüche 1 oder 2, dadurch gekennzeichnet, daß die Kontroll-Sequenz der Transcription eine natürliche oder synthetische, vollständige oder unvollständige palindromische Sequenz ist.

4. Verfahren nach Anspruch 3, dadurch gekennzeichnet, daß die genannte palindromische Sequenz mehrmals wiederholt wird.

5. Verfahren nach einem der Ansprüche 1 bis 4, dadurch gekennzeichnet, daß der Rezeptor ein nuklearer Rezeptor ist und der Ligand die entsprechende spezifische Verbindung ist.

6. Verfahren nach Anspruch 5, dadurch gekennzeichnet, daß der genannte Rezeptor der Rezeptor von Östrogen ist, der genannte Ligand ein Östrogen ist und der essentielle Teil, der den Liganden erkennt, vom Rezeptor hER abstammt.

7. Verfahren nach Anspruch 5, dadurch gekennzeichnet, daß der essentielle Teil des Rezeptors, der den Liganden erkennt, vom humanen Östrogen-Rezeptor abstammt, bezeichnet als hERG, wobei der Ligand ein Östrogen ist.

8. Verfahren nach Anspruch 7, dadurch gekennzeichnet, daß der Rezeptor der Rezeptor hERG ist und der Ligand Östradiol ist.

9. Verfahren nach einem der Ansprüche 7 oder 8, dadurch gekennzeichnet, daß das Östradiol in einer Konzentration zwischen 2 und 50 nM, vorzugsweise in der Größenordnung von 10 nM, zugesetzt wird.

10. Verfahren nach einem der Ansprüche 1 bis 9, dadurch gekennzeichnet, daß sich die DNA-Sequenz in der Hefe, die für das genannte Protein unter Kontrolle von Elementen codiert, die seine Expression in den Hefen und/oder der DNA-Sequenz gewährleisten, die für den genannten Rezeptor codiert, auf einem Plasmid befindet, das einen Anfang der funktionellen Replikation in der Hefe umfaßt.

11. Verfahren nach Anspruch 10, dadurch gekennzeichnet, daß sich die beiden DNA-Sequenzen auf dem gleichen Plasmid befinden.

12. Verfahren nach einem der Ansprüche 1 bis 11, dadurch gekennzeichnet, daß die funktionelle DNA-Sequenz in der Hefe, die für den genannten Rezeptor codiert, durch ein Chromosom der Hefe getragen wird.

13. Verfahren nach einem der Ansprüche 1 bis 12, dadurch gekennzeichnet, daß man einen Hefestamm mit einem Defizit an Proteasen verwendet.

14. Verfahren nach einem der Ansprüche 1 bis 13, dadurch gekennzeichnet, daß der Ligand am Ende der Stufe des Zellwachstums zugesetzt wird.

15. Expressions-Vektor, induzierbar von einem bestimmten Protein in Hefezellen, die durch den genannten Vektor transformiert wurden, dadurch gekennzeichnet, daß er umfaßt:
- eine DNA-Sequenz, die für das genannte Protein unter der Kontrolle von Elementen codiert, die seine Expression in den Hefen gewährleisten, wobei die genannten Elemente umfassen:
- eine Kontroll-Sequenz der Transcription vom Ursprung höherer Eucaryoten, umfassend eine natürliche Aktivator-Sequenz "HRE", eine Variante oder ein Derivat von dieser, induzierbar durch einen von einem Rezeptor und seinem Liganden gebildeten Komplex,
wobei die genannten Hefezellen eine funktionelle DNA-Sequenz in der Hefe umfassen, die für den genannten Rezeptor codiert, der ein natürlicher nuklearer Rezeptor ist, ausgewählt unter den Rezeptoren für ein Steroid, den Rezeptoren für Retinoide, für Thyroid-Hormone und für Vitamin D3, einer Rezeptor-Variante oder einer Rezeptor-Chimäre, für die Durchführung des Verfahrens nach einem der Ansprüche 1 bis 14.

16. Vektor nach Anspruch 15, dadurch gekennzeichnet, daß die DNA-Sequenz, die für den genannten Rezeptor codiert, durch den induzierbaren Expressions-Vektor getragen wird.

17. Expressions-Vektor nach einem der Ansprüche 15 oder 16, dadurch gekennzeichnet, daß die Kontroll-Sequenz der Transcription vom Ursprung höherer Eucaryoten sensibel für den Komplex ist, der durch einen Steroid-Rezeptor und das entsprechende Steroid gebildet wird.

18. Expressions-Vektor nach einem der Ansprüche 15 bis 17, dadurch gekennzeichnet, daß die Kontroll-Sequenz der Transcription vom Ursprung höherer Eucaryoten sensibel für den Komplex ist, der durch den Östradiol-Rezeptor und Östradiol gebildet wird.

19. Expressions-Vektor nach einem der Ansprüche 15 bis 18, dadurch gekennzeichnet, daß die Kontroll-Sequenz der Transcription vom Ursprung höherer Eucaryoten die Sequenz oder deren Wiederholungen von -605 bis -634 des Gens vom Vitellogenin des Hühnchens umfaßt.

20. Expressions-Vektor nach Anspruch 19, bestehend aus dem Plasmid pYERE3/hER, hinterlegt bei CNCM unter der Nummer I-770.

21. Hefestämme, transformiert durch den Expressions-Vektor nach einem der Ansprüche 15 bis 20.
